# EUROPEAN PATENT APPLICATION

(11) **EP 3 127 499 A1**
(43) Date of publication of application: **08.02.2017**
(21) Application number: 15180088.5
(22) Date of filing: 06.08.2015
(51) Int. Cl.: A61B 17/70

(54) **INSTRUMENT FOR CORRECTING A POSITION OF ONE OR MORE VERTEBRAE OF THE SPINE**

(71) Applicant: Biedermann Technologies GmbH & Co. KG, 78166 Donaueschingen (DE)
(72) Inventor: Matthis, Wilfried, 79367 Weisweil (DE); Dannecker, Berthold, 78112 St. Georgen (DE); Biedermann, Timo, 78647 Trossingen (DE)
(74) Representative: Prüfer & Partner mbB Patentanwälte · Rechtsanwälte

(57) **Abstract**

An instrument for correcting a position of one or more vertebrae of a spine is provided, the instrument including
a first implant extension (1) having a first end portion (1a) configured to releasably engage a vertebral implant, a second end portion (1b) and a longitudinal axis (21) extending between the first end portion and the second end portion,
a second implant extension (2) having a first end portion (2a) configured to releasably engage a vertebral implant and a second end portion (2b) and a longitudinal axis (22) extending between the first end portion and the second end portion,
a connecting device (5) for connecting the first implant extension (1) and the second implant extension (2);
wherein the connecting device (5) comprises a first clamping member (6) configured to be fixed to the first implant extension (1) and a second clamping member (7) configured to be fixed to the second implant extension (2) and wherein the first clamping member (6) is connected to the second clamping member (7) through a connecting portion (8) and wherein the connecting portion (8) and the first and second clamping members (6, 7) are stnictured and arranged to allow polyaxial orientation between the first clamping member (6) and the connection portion (8) and between the second clamping member (7) and the connection portion (8);
and wherein at least the first clamping member (6) is mountable onto the first implant extension (1) from the second end (1b) thereof.

## Description

The invention relates to an instrument for correcting the position of one or more vertebrae of the spine. The instrument includes a first and a second implant extension configured to be connected to a spinal implant and a connecting device for connecting the first and second implant extensions. The connecting device comprises a first and a second clamping member configured to be fixed to the first and second implant extensions, respectively, and being connected by a connecting portion. With the connecting device the first and second implant extensions can be fixed relative to each other in a multitude of orientations.

A system for correcting aligmnent of one or more vertebrae of a spine is known, for example, from US 2011/0106082 A1. The system includes at least two elongated extensions mounted to and extending proximally from at least one vertebral body. A linking assembly extends between and links proximal ends of the extensions to one another. The linking assembly includes first and second mounting assemblies movably engaged to an elongate link member extending transversely to the extensions. Each of the mounting assemblies includes a movable joint for receiving the proximal end of a respective one of the extensions and a cam assembly for securing the mounting assembly in position relative to the link member and to fix the joint around the extension.

US 7,276,069 B2 describes a connecting element for connecting two rods or screws used for bone or vertebra stabilization. The connecting element comprises a connecting portion, a first portion which can be connected to one of the rods and a second portion which can be connected to the other rod. The first portion is connected to the connecting portion by a device allowing polyaxial orientation to permit fixation of rods that are inclined at an angle or that are oblique relative to each other.

It is the object of the invention to provide an instrument for correcting a position of one or more vertebrae of a spine that has a simple design and is easy to handle. Furthermore, a system comprising such an instrument and a spinal stabilization device shall be provided. Lastly, a connecting device for use with implant extensions to form such an instrument shall be provided.

The object is solved by an instrument according to claim 1, by a system according to claim 16 and a connecting device of claim 17. Further developments are given in the dependent claims.

The first and second implant extensions are connectable to a spinal implant, such as a bone anchor and preferably to a pedicle screw. The connecting device is easily mountable to the implant extensions. For mounting, the connecting device can be placed over the free ends of the implant extensions by holding the connecting device with one or both hands. Due to the polyaxial connection of the connecting portion with each of the clamping members the alignment of the clamping members with the implant extensions is facilitated, in particular in cases where the implant extensions are extending at an angle relative to each other or are located offset with respect to each other on one vertebra.

The fixation of the first and second clamping member can be effected by manually actuating a handle portion of the instrument in a first direction. Similarly, the clamping members can be loosened relative to the implant extensions by actuating the handle portion in an opposite direction. Hence, the connecting device can be quickly mounted and dismounted without using a separate tool. This renders a surgical procedure, in particular a multi-segmental procedure of correcting spinal deformities more efficient.

The handle portion may have a shape and/or a structure that facilitates gripping and allows to transmit sufficient forces so as to pull the implant extension by pulling at the handle portion. Thereby, a position of a vertebra that is connected to the implant can be corrected.

In a further aspect, the length of the connecting element can be adjusted. This enlarges the variety of applications of the instrument.

With the implant extensions in the form of a sleeve or tube the instrument is particularly suitable for minimally invasive and minimally open surgery. The connecting device can be used with existing implant extensions.

Further features and advantages will become apparent from the description of embodiments by means of the accompanying drawings. In the drawings:
- Fig. 1: shows a perspective view of the instrument according to an embodiment.
- Fig. 2: shows a perspective exploded view of the connecting device of the instrument of Fig. 1
- Fig. 3: shows a cross-sectional view of a portion of the instrument including the connecting portion and a portion of the implant extensions as shown in Fig. 1, the cross-section being taken in a plane extending through a longitudinal axis of the implant extensions and a central axis of the connecting device perpendicular to the longitudinal axis.
- Fig. 4: shows a perspective view of the application of the instrument to pedicle screws inserted into the pedicles of a spinal column.
- Fig. 5: shows a front perspective view of a clamping member of the instrument shown in Figs. 1 to 4.
- Fig. 6: shows a rear perspective view of the clamping member of Fig. 5
- Fig. 7: shows a side view of the clamping member of Figs. 5 and 6.
- Fig. 8: shows a cross-sectional view of the clamping member of Figs. 5 to 7, the cross-section taken a long line A-A of Fig. 7
- Fig. 9: shows a front perspective view of a handle portion of the instruments Figs. 1 to 4.
- Fig. 10: shows a cross-sectional view of the handle portion of Fig. 9, the cross-section taken in a plane comprising the longitudinal axis of the handle portion.
- Fig. 11: shows a perspective view of the connection portion of the connecting member of Figs. 1 to 4.
- Fig. 12: shows a cross-sectional view of the connecting portion of Fig. 11, the cross-section taken in a plane including the central axis of rotation of the connecting portion.
- Fig. 13: shows a perspective view from the top of a pressure member of the connecting portion of Figs. 1 to 4.
- Fig. 14: shows a perspective view from the bottom of the pressure member of Fig. 13.
- Fig. 15: shows a cross-sectional view of the pressure member of Figs. 13 and 14, the cross-section taken in a plane including the central axis and extending perpendicular to a cylindrical support surface.
- Figs. 16a to 16c: show steps of mounting the instrument to spinal implants in a form of pedicle screws.
- Figs. 17a to 17c: show perspective views of positions of the implant extensions relative to each other when connected through the connecting member.

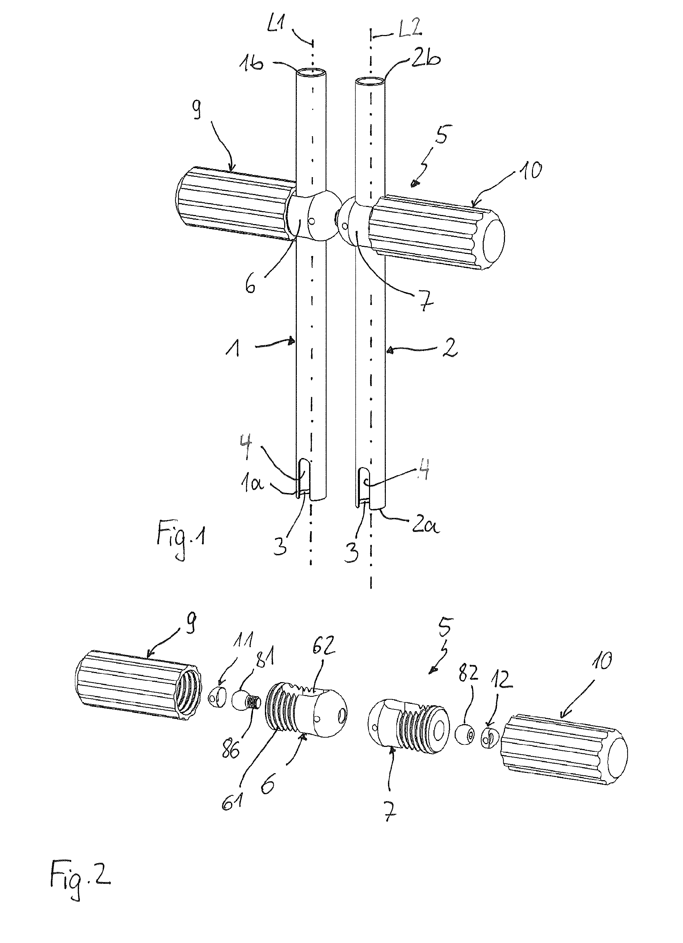

Referring to Figs. 1 to 4, the instrument includes a first implant extension 1 and a second implant extension 2 configured to be releasably connected to a spinal implant. The implant extensions 1, 2 may be elongated tubes having a front end 1a, 2a and a rear end 1b, 2b and a central longitudinal axis L1, L2. Each implant extension 1, 2 comprises at the front end 1a, 2a an engagement portion 3 for engaging the spinal implant, such as, for example, a spinal bone anchor 100 anchored in a vertebra 300. The engagement portion 3 may be, for example, a projection at an inner wall of the respective implant extension that is configured to engage a groove at the spinal implant, for example, a groove at an outer wall of a receiving part of a bone anchor. An inner diameter of the implant extension is such that the implant extension can be placed over the spinal implant and attached thereto. Furthermore, each implant extension 1, 2 may comprise a substantially U-shaped recess 4 that has a size such that the implant extension can be placed over a spinal rod 200 extending through the receiving part of a bone anchor. An inner diameter of the entire implant extensions 1, 2 may be sized so as to allow insertion of a tool or a part of the implant therethrough. For example, a locking element such as a set screw may be inserted through the implant extensions.

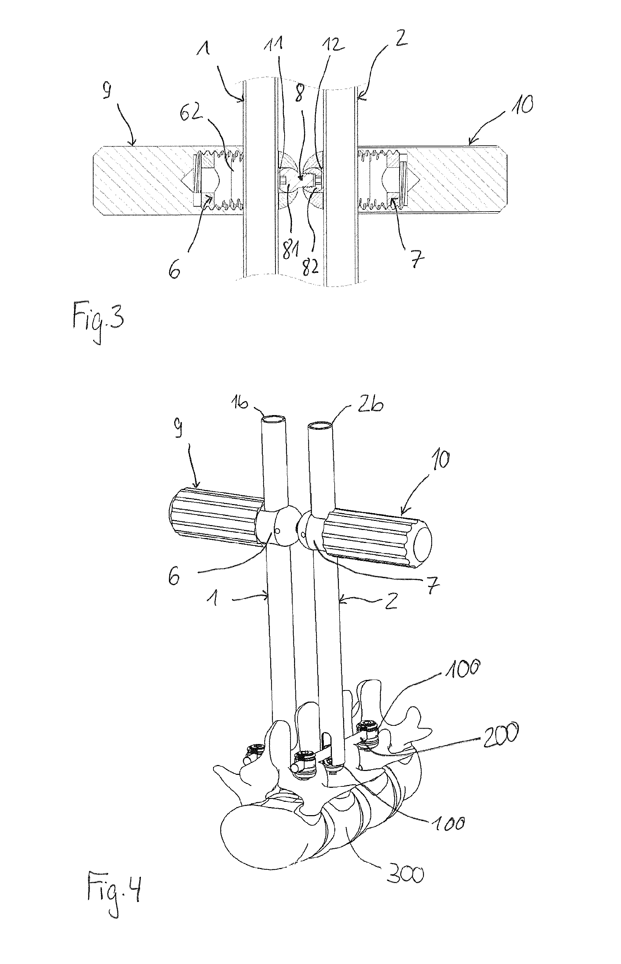

For connecting the first implant extension 1 to the second implant extension 2 a connecting device 5 is provided. The connecting device 5 includes a first clamping member 6 configured to be connected to the first implant extension 1 and a second clamping member 7 configured to be connected to the second implant extension 2. The first clamping member 6 and the second clamping member 7 are connected through a connecting portion 8, as depicted in particular in Fig. 3, that is structured and arranged to allow a polyaxial orientation between the connecting portion 8 and the first clamping member 6 and the second clamping member 7 respectively. To fix the position of the first clamping member 6 on the first implant extension 1 a first handle portion 9 is provided that is connected to the first clamping member 6 and can be actuated in a first direction to advance on the first clamping member 6 thereby exerting a pressure onto the first implant extension 1. Similarly, the first handle portion 9 can be actuated in a second direction opposite to the first direction to release the pressure onto the first implant extension 1 to loosen the fixation of the clamping member 6. Symmetrically, a second handle portion 10 is provided that is connected to the second clamping member 7 to fix the second clamping member 7 to the second implant extension 2. The second handle portion 10 can be actuated in a first direction to advance on the second clamping member 7 thereby exerting pressure onto the second implant extension 2 and can be actuated in the opposite direction to release the pressure from the second implant extension 2 to loosen it. In order to transmit the pressure from the handle portions 9, 10 to the connecting portion 8 first and second pressure elements 11, 12 are provided that are configured to be arranged between each implant extension 1, 2 and the connecting portion 8 in the clamping device, 6, 7. Hence, by means of the pressure resulting from actuating the handle portions 9, 10, also pressure can be exerted onto the connecting portion 8 to lock the connecting portion 8 in the respective clamping member 6, 7 wherein the whole instrument is locked.

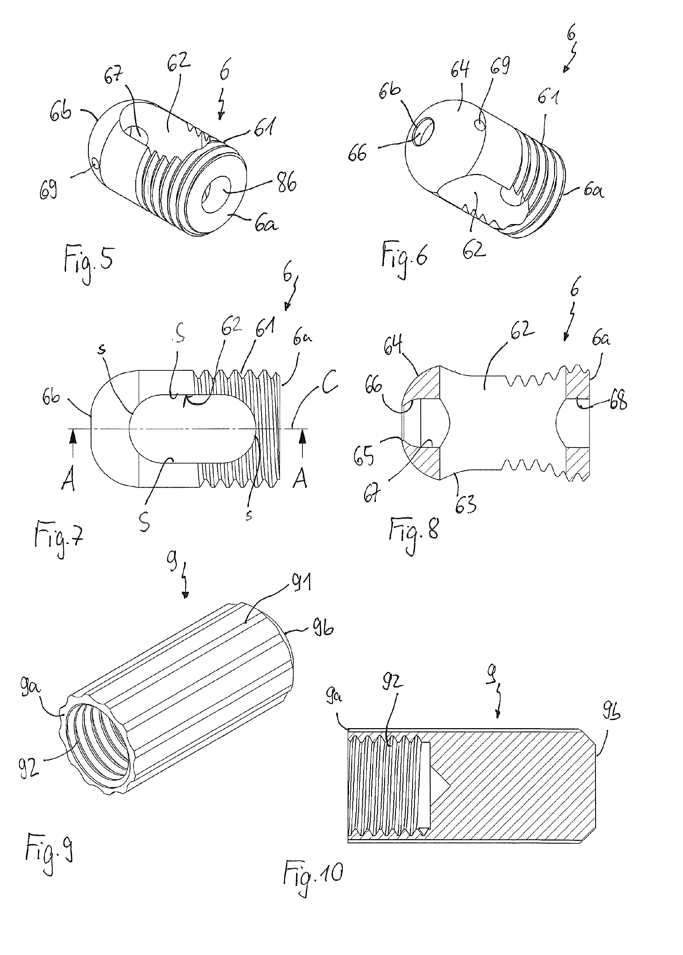

The clamping members 6, 7 will be described referring more in detail to Figs. 5 to 8 which show the first clamping member 6. The second clamping member 7 is identical to the first clamping member 6 and will not be described separately. The clamping member 6 is a substantially cylindrical part with a front end 6a and a rear end 6b and an outer thread 61 adjacent to the front end 6a that extends in an axial direction up to a distance from the front end 6a. A recess 62 extends completely through the first clamping member 6 from one side to an opposite side of the clamping member 6. The recess 62 is substantially oblong with opposite long sides a S extending substantially parallel to a central axis C of the clamping member 6 and connecting short sides s. The long sides S are substantially straight and the short sides s are cylinder segment-shaped with a radius adapted to guide the first implant extension 1 therein. A total length of the recess 62 is greater than an outer diameter of the first implant extension 1 such that when the first implant extension 1 is guided through the recess 62 it can also move slightly in direction transverse to its longitudinal axis along the central axis C of the clamping member 6. The oblong recess 62 forms a channel for receiving the implant extension 1.

An outer shape of a portion 64 of the first clamping member 6 in a region adjacent to the rear end 6b up to an axial position of an end region of the oblong recess 62 is a substantially spherically shaped. This permits pivoting of the first clamping member 6 and the second clamping member 7 relative to each other without obstacle. The clamping member 6 comprises at its rear end 6b an opening 65 that opens into a seat portion 66 which continues into a hollow cylindrical portion 67 that is in communication with the oblong recess 62. The seat portion 66 and the cylindrical portion 67 provide an accommodation space for the connecting portion 8 and the pressure element 11 as described below. In particular, the seat portion 66 is shaped to allow pivoting of the connecting portion therein in a plurality of directions, i.e. to allow polyaxial movement of the connecting portion 8 with respect to the clamping member 6. The seat portion 66 may include a spherical segment-shaped inner surface portion but is not limited thereto. Furthermore, an opening 68 extends from the front end 6a through the clamping member into the oblong recess 62. The opening 68 serves for inserting the connection portion and the pressure element 11 and may serve also for inserting a tool for holding the connecting portion.

Turning to Figs. 9 and 10, the first handle portion 9 will be described. The second handle portion 10 is identical to the first handle portion 9. The first handle portion is a substantially cylindrical part that may have a length adapted to be gripped with at least two fingers preferably with three or four fingers in a row along a longitudinal direction of the handle portion. This means, when the clamping device 6 is fixed to the implant extension 1, a person is able to grip the handle portion 9 and move the vertebra attached via the bone anchor to the extension device 1 by pulling or pressing via the handle portion 9. More specifically, the handle portion 9 has a front end 9a and an opposite rear end 9b. In at least an outer surface portion a gripping structure 91, such as longitudinal ribs or similar structures may be provided. An outer diameter of the handle portion is larger, preferably only slightly larger than an outer diameter of the clamping device 6, as depicted in Fig. 3.

A threaded bore 92 extends from the front end 9a to a distance from the front end for screwing in the first clamping member 6.

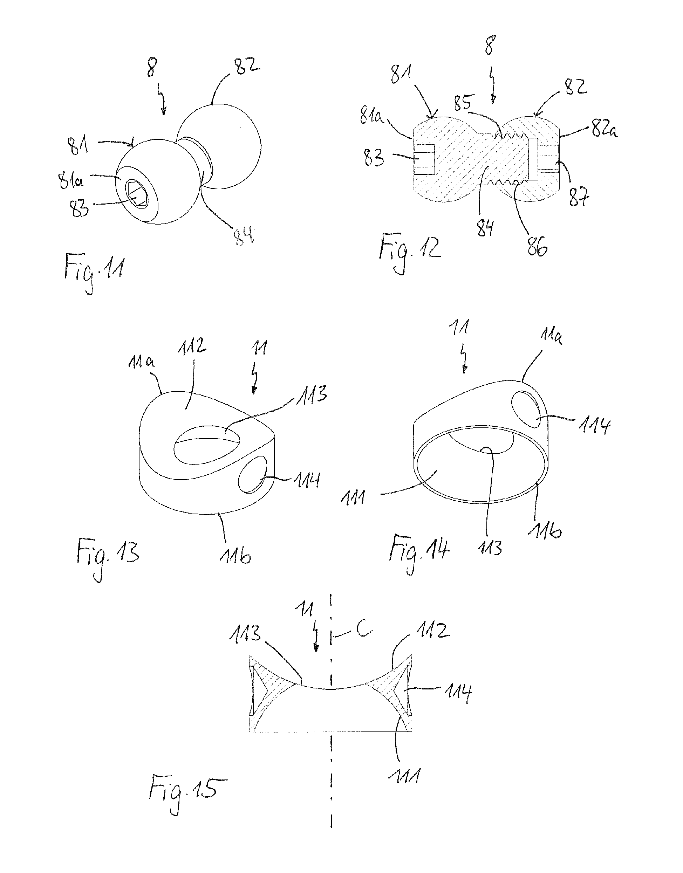

Turning to Figs. 11 and 12, the connecting portion 8 comprises a first head 81 for connecting the connection portion 8 to the first clamping member 6 and a second head 82 for connecting the connection portion 8 to the second clamping member 7. The first head 81 has a spherical segment-shaped outer surface portion and a free end 81a with a recess 83 for engagement with the screwing-in tool. Opposite to the free end 81a a neck portion 84 is provided that comprises an external thread 85. The second head 82 also comprises a spherical segment-shaped outer surface portion and a free end 82a that faces in the opposite direction of the free end 81a of the first head 81 when the heads are connected. At its side opposite to the free end 82a a threaded bore 86 is provided in the second head 82 into which the neck portion 84 can be screwed to connect the two heads. Also, the second head 82 comprises an engagement recess 87 for engagement with a tool. The length of the neck portion 84 may be such that in the mounted condition there is only a small distance between the two clamping members 6, 7 as depicted in Fig. 3. The opening 65 of the first clamping member 6 is smaller than a largest outer diameter of the heads 81, 82 of the connecting portion such that the heads cannot be removed through the opening 65 of the clamping device 6.

Referring to Figs. 13 to 15, the first pressure element 11 will be described. The second pressure element 12 is identical to the first pressure element. The first pressure element 11 is a substantially cylindrical part with an outer diameter that is sized so that the pressure element can be placed into the cylindrical section 67 of the accommodation space of the clamping member 6. The pressure element 11 has an upper end 11a and an opposite lower end 11b. Adjacent to the lower end 11b, a substantially spherical recess 111 is formed with a shape matching the outer shape of the first head 81 for distributing pressure onto the first head 81. Adjacent to the first end 11a, a substantially cylinder segment-shaped recess 112 is provided the size and shape of which is adapted to support the first implant extension 1 when the first implant extension 1 is inserted into the oblong recess 62 of the first clamping member 6.

A coaxial hole 113 extends through the pressure element to allow access with a tool through the pressure element 11 to the head 81. Furthermore, the pressure element 11 comprises crimp bores 114 in its outer surface on both sides of the cylindrical recess 112 that cooperate with crimp bores 69 at the first clamping member 6 to inhibit rotation of the pressure element 11 once it is inserted into the first clamping member 6 to keep the cylindrical recess 112 of the pressure element 11 aligned with the bottom of the oblong recess 62.

The first and second implant extensions 1, 2 may be made of a bio-compatible material, for example of titanium or stainless steel, a bio-compatible alloy, such as a NiTi-alloy, for example Nitinol, magnesium or magnesium alloys or from a bio-compatible plastic material, such as, for example, polyether ether ketone (PEEK) or poly-1-lactide acid (PLLA). The parts of the connecting device 5 may be made of the same material as the implant extensions or of a different material. In particular, the parts of the connecting device need not to be made of a bio-compatible material as the connecting device usually is not in contact with the patient's body.

The connecting device 5 is mounted as follows. The first head 81 is inserted through the opening 68 into the first clamping member 6 until it rests in the seat 66 and the neck portion 84 extends through the opening 65. Then the pressure element 11 is inserted through the opening 68 at the front end 6a into the cylindrical section 67 of the first clamping member 6 so that its spherical recess 111 faces the first head 81. The second head 82 and the second pressure element 12 are mounted in the same manner to the second clamping device 7. Then, the first head 81 and the second head 82 are connected by screwing the neck portion 84 into the threaded bore 86. This may be done using instruments (not shown). Finally, the handle portions 9 and 10 are screwed onto the threaded outer surface of the first clamping member 6 and of the second clamping member 7, respectively. The oblong recess 62 in each clamping member may be only slightly covered by the first and second handle portions 9, 10, respectively such that there is ample space to insert the first and second implant extensions 1, 2, respectively. This facilitates the placement of the connecting device 5 onto the implant extensions.

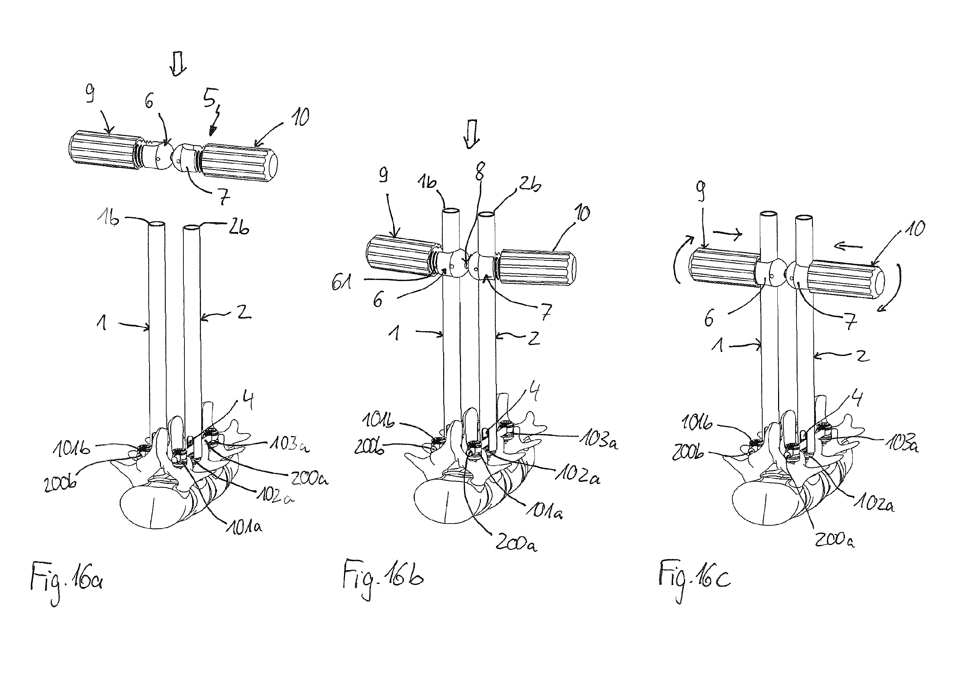

Operation will be described with reference to Figs. 16a to 16c. Preferably, the instrument is used in a case, where a middle vertebra between two vertebrae has to be pulled up to be connected to a spinal rod. However, the use of the instrument is not limited thereto and other applications may be contemplated. As shown in Fig. 16a, three bone anchors 101a, 101b, 101c are placed in three adjacent vertebrae on one side of the vertebrae and three bone anchors 101b, 102b, 103b (not shown) are placed on the other side of the vertebrae. A first spinal rod 200a extends to the bone anchors on the one side and a second spinal rod 200b extends through the bone anchors on the other side. The outer bone anchors 101a, 103a, 101b, 103b are each fixed to the spinal rod while the bone anchors 102a, 102b (not shown) in the middle vertebra are not yet fixed to the spinal rods. As depicted in Fig. 16a, the first implant extension 1 is mounted to the middle bone anchor 102a and the second implant extension 2 is mounted to the middle bone anchor 102b on the opposite side.

Then, as shown in Fig. 16b, the connecting device 5 is placed onto the implant extensions 1, 2 by inserting the free second ends 1b, 2b of the implant extensions 1, 2 into the oblong recesses 62 of the clamping devices 6, 7, respectively. The heads 81, 82 are polyaxially pivotable in the clamping devices 6, 7 such that the clamping devices 6, 7 can be oriented with respect to the implant extensions 1, 2 and with respect to each other in a multiplicity of directions.

Once the angular position of the heads 81, 82 in the connecting device 5 has been adjusted, the handle portions 9, 10 are tightened so that their respective front portions press onto the implant extensions 1,2 until the clamping devices 6,7 are fixed to the implant extensions 1, 2. Simultaneously, the implant extensions 1, 2 each press onto the pressure elements 11, 12 so that the pressure is transferred to the heads 81, 82. Thereby, the angular position of the heads 81, 82 is locked and as a result, the whole instrument is locked. The vertebra associated with the middle bone anchors 102a, 102b can be pulled upward towards the respective spinal rod 200a, 200b such that the spinal rod is placed correctly in the bottom of a channel of the bone anchor (not shown). In this position, a locking element (not shown) may be inserted through the implant extensions 1, 2, respectively until it is received in the receiving part of the respective bone anchor. Finally, the locking element may be tightened using an instrument that is configured to extend through the implant extensions 1, 2.

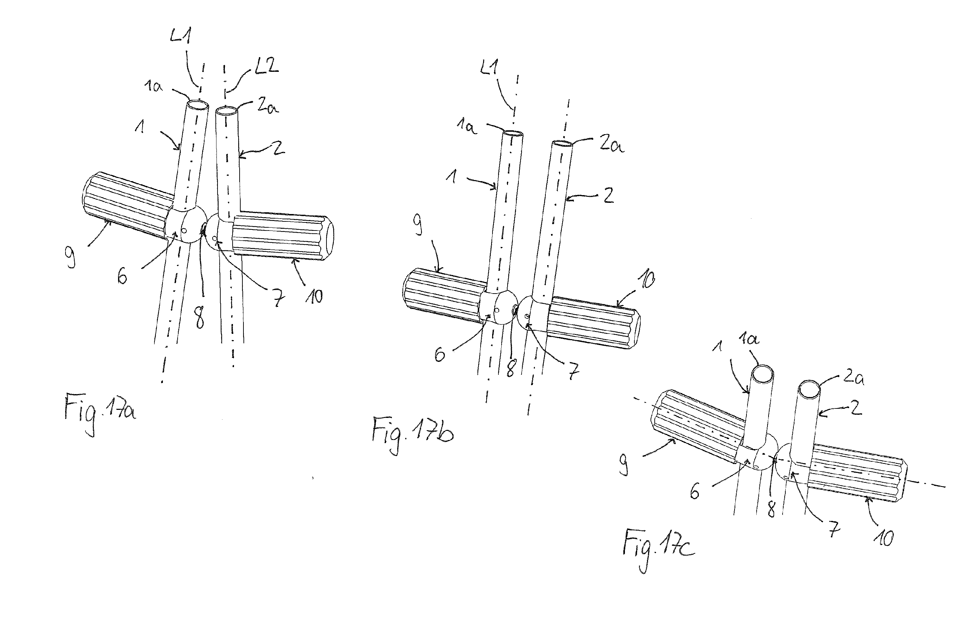

Figs. 17a to 17c illustrate that the mounting and the fixation of the connecting device 5 can be carried out also in the case of non-parallel or axially displaced implant extensions. In Fig. 17a, the implant extensions 1, 2 are oriented towards each other in the direction of their free ends 1b, 2b. The connecting device 5 can be mounted by pivoting the clamping members 6, 7 relative to the heads 81, 82 in the upward direction towards the free ends 1b, 2b of the implant extensions 1, 2. In Fig. 17b the implant extensions 1, 2 are oriented slightly away from each other towards their free ends 1b, 2b. The connecting device 5 can be mounted by pivoting the clamping members relative to the heads towards the front end 1a, 2a of the implant extensions 1, 2. In Fig. 17c the implant extensions 1, 2 are slightly axially offset from each other. The connecting device 5 can be mounted by pivoting one clamping member relative to the other towards the axially off-set implant extension.

Modifications of the instrument may be contemplated. For example, the implant extensions can have another shape. They need not to be tubular shaped but can also be in the form of any rod-like element that is connectable to a spinal implant or a portion thereof. The implant extension devices need not to be mounted to different implants, such as different bone screws. It may also be contemplated that the front portion of the implant extension is structured and arranged to engage a spinal rod and that the implant extensions are connected at different positions to one single spinal rod.

For the engagement mechanism of the implant extension with the spinal implant, any other engagement mechanism, such as, for example, a threaded engagement with the spinal implant may be contemplated.

The shape of heads of the connecting portion and the seat portion in the clamping member may be different from the embodiment shown. Any shape that allows a pivoting movement can be used, for example, the seat may be conically shaped. The pressure elements may be omitted.

It may be contemplated that the length of the connecting portion can be adjusted. For example, the neck portion can be made longer and a length adjustment structure for the connection of the first head to the second head may be provided.

While the instrument has been shown to have a symmetrical design, the first and second implant extensions may be different. Also the first and second clamping members can be shaped differently. For example, one clamping member may have a right hand thread and the other clamping member may have a left hand thread. The handle portions may have a corresponding thread. This would allow to apply a symmetrical movement of a user's hands when tightening or loosening the clamping members.

## Claims

1. Instrument for correcting a position of one or more vertebrae of a spine, the instrument including
a first implant extension (1) having a first end portion (1a) configured to releasably engage a vertebral implant, a second end portion (1b) and a longitudinal axis (L1) extending between the first end portion and the second end portion,
a second implant extension (2) having a first end portion (2a) configured to releasably engage a vertebral implant and a second end portion (2b) and a longitudinal axis (L2) extending between the first end portion and the second end portion,
a connecting device (5) for connecting the first implant extension (1) and the second implant extension (2);
wherein the connecting device (5) comprises a first clamping member (6) configured to be fixed to the first implant extension (1) and a second clamping member (7) configured to be fixed to the second implant extension (2) and wherein the first clamping member (6) is connected to the second clamping member (7) through a connecting portion (8) and wherein the connecting portion (8) and the first and second clamping members (6, 7) are structured and arranged to allow polyaxial orientation between the first clamping member (6) and the connection portion (8) and between the second clamping member (7) and the connection portion (8);
and wherein at least the first clamping member (6) is mountable onto the first implant extension (1) from the second end (1b) thereof.

2. The instrument of claim 1, wherein the second clamping member (7) is mountable onto the second implant extension (2) from the second end (2b) thereof

3. The instrument of claim 1 or 2, wherein the first clamping member (6) comprises a channel (62) to receive the first implant extension (1) and wherein the channel (62) is closed in such a manner that the first implant extension can be inserted and removed only in the direction of the longitudinal axis (L1).

4. The instrument of one of claims 1 to 3, wherein at least a first handle portion (9) is provided that is connectable to the first clamping member (6) and that is configured to be manually actuated so as to lock the first implant extension (1) in the first clamping member (6).

5. The instrument of claim 4, wherein the first handle portion is also configured to be actuated to lock the orientation of the first clamping member (6) relative to the connection portion (8).

6. The instrument of claim 4 or 5, wherein when the first handle portion (9) is connected to the first clamping member (6) and actuated in a first direction it is configured to exert an increasing pressure onto the first implant extension (1) and when it is actuated in a second direction opposite to the first direction the pressure onto the first implant extension (1) decreases.

7. The instrument of one of claims 1 to 6, wherein the second clamping member (7) comprises a channel to receive the second implant extension (2) and wherein the channel is closed in such a manner that the second implant extension (2) can be inserted and removed only in the direction of the longitudinal axis.

8. The instrument of one of claims 1 to 7, wherein a second handle portion (10) is provided that is connectable to the second clamping member (7) and that is configured to be manually actuated so as to lock the second implant extension (2) in the second clamping member (7).

9. The implant extension of claim 8, wherein the second handle portion (10) is also configured to be actuated to lock the orientation of the second clamping member (7) relative to the connection portion (8).

10. The instrument of claim 8 or 9, wherein when the second handle portion (10) is connected to the second clamping member (7) and actuated in a first direction it is configured to exert an increasing pressure onto the second implant extension (2) and when it is actuated in a second direction the pressure onto the second implant extension (2) decreases.

11. The instrument of one of claims 1 to 10, wherein the connecting portion (8) comprises a first head (81) and the first clamping member (6) has a receiving portion (66) for pivotably receiving the first head (81) and wherein the connecting portion (8) comprises a second head (82) and the second clamping member (7) comprises a receiving portion for pivotably receiving the second head (82).

12. The instrument of claim 11, wherein the first head (81) and the second head (82) comprise a spherically shaped outer surface portion.

13. The instrument of claim 11 or 12, wherein the first head (81) and the second head (82) are connected through a rod-like portion (84).

14. The instrument of one of claims 11 to 13, wherein a first pressure member (11) is provided that is configured to be arranged in the first clamping member (6) between the first head (81) and the first implant extension (1) and wherein a second pressure member (12) is provided that is configured to be arranged in the second clamping member (7) between the second head (82) and the second implant extension (2) for exerting pressure onto the first head (82) and the second head, respectively.

15. The instrument of one of claims 1 to 14, wherein the first end portions (1a, 1b) of the first and second implant extensions (1, 2) are configured to be connected to a receiving part of a pedicle screw and each have a recess (4) for passing through a spinal rod and wherein preferably the implant extensions are tube-shaped.

16. A system of an instrument of one of claims 1 to 15 and a first spinal implant, preferably a bone anchor, connectable to the first implant extension (1) and a second spinal implant, preferably a bone anchor, connectable to the second implant extension (2).

17. A connecting device for connecting at least two implant extensions, the connecting device including a first clamping member (6) configured to be fixed to a first elongate implant extension (1) having a free end (1b) and a second clamping member (7) configured to be fixed to a second elongate implant extension (2) having a free end (2b) and wherein the first clamping member (6) is connected to the second clamping member (7) through a connecting portion (8) and
wherein the connecting portion (8) and the first and second clamping members (6, 7) are structured and arranged to allow polyaxial orientation between the first clamping member (6) and the connection portion (8) and between the second clamping member (7) and the connection portion (8), and
wherein at least the first or the second clamping member (6, 7) comprises a channel (62) for receiving the first or the second implant extension (1, 2) wherein the channel is shaped such that the first or second second implant extension (1, 2) can be inserted into the channel only from the free end (1b, 2b) thereof.
